# EUROPEAN PATENT SPECIFICATION

(11) **EP 3 309 158 B1**
(45) Date of publication and mention of the grant of the patent: **16.03.2022**
(21) Application number: 17186150.3
(22) Date of filing: 19.12.2013
(51) Int. Cl.: C07D 413/14, A61K 31/422, A61P 7/00

(54) **CRYSTALLINE FORM K OF RIVAROXABAN AND PROCESS FOR ITS PREPARATION**
KRISTALLINE FORM K VON RIVAROXABAN AND VERFAHREN ZUR HERSTELLUNG
FORME CRISTALLINE K DU RIVAROXABAN ET PROCÉDÉ DE PRÉPARATION

(30) Priority: 21.12.2012 SI 201200386
(43) Date of publication of application: 18.04.2018
(62) Divisional of application: 13811529.0
(73) Proprietor: Krka, tovarna zdravil, d.d., 8501 Novo mesto (SI)
(72) Inventor: Zupancic, Silvo, 8000 Novo mesto (SI); Benkic, Primoz, 1000 Ljubljana (SI)
(74) Representative: Uexküll & Stolberg

(56) References cited:
- WO-A1-2010/075631
- US-A1- 2010 152 189
- CAIRA ED - MONTCHAMP JEAN-LUC: "Crystalline Polymorphism of Organic Compounds", TOPICS IN CURRENT CHEMISTRY; [TOPICS IN CURRENT CHEMISTRY], SPRINGER, BERLIN, DE, vol. 198, 1 January 1998 (1998-01-01), pages 163-208, XP008166276, ISSN: 0340-1022

## Description

The present invention relates to new polymorphic form K of rivaroxaban, a process of its crystallization, its use for the formulation of a pharmaceutical dosage form and a pharmaceutical dosage form comprising it.

### Background of the invention

Rivaroxaban is the INN of the anticoagulant compound 5-chloro-N-{[(5S)-2-oxo-3-[4-(3-oxomorpholin-4-yl)phenyl]oxazolidin-5-yl]methyl}thiophene-2-carboxamide, which was disclosed in WO 01/47919. Rivaroxaban is a small molecule inhibitor of blood coagulation factor Xa and is used in the prophylaxis and treatment of thromboembolic diseases such as heart attack, angina pectoris, reocclusion and restenosis following angioplasty or bypass, cerebral apoplexy, transient ischemic attack, peripheral arterial obstructive diseases, pulmonary embolism and venous thrombosis.

Rivaroxaban corresponds to the formula (I):

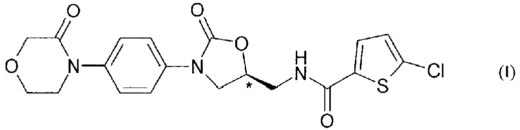

A process for the preparation of rivaroxaban, intermediates of rivaroxaban, and their preparation is disclosed for example in WO 01/47919 (Example 44). This document discloses the synthesis of rivaroxaban from the intermediate 2-({(5*S*)-2-Oxo-3-[4-(3-oxo-4-morpholinyl)phenyl]-1,3-oxazolidin-5-yl}methyl)-1*H-*isoindol-1,3(2*H*)-dion.

The process is illustrated in the following scheme:

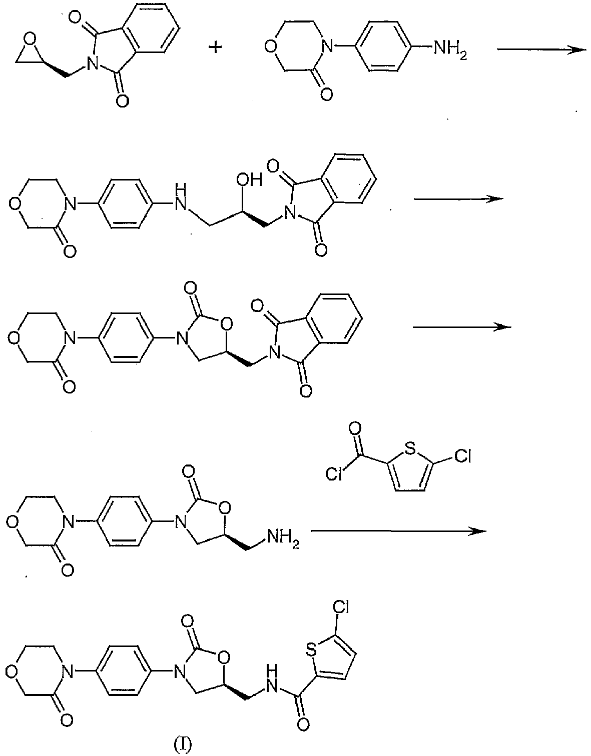

This process has various disadvantages in the reaction management which has particularly unfavorable effects for preparation of the rivaroxaban on the industrial scale. Furthermore, low yields and the need to purify rivaroxaban by tedious chromatographic purification, i.e. by flash-chromatography from mixture dichloromethane/methanol, makes this process not suitable for the synthesis of rivaroxaban at industrial scale.

A similar process is described also in Journal of Medicinal chemistry, 2005, 48, 5900-5908 and DE 10129725.

WO 2004/060887 discloses a method for producing rivaroxaban from 5-chlorothiophene-2-carbonyl chloride, (2*S*)-3-aminopropane-1,2-diol and 4-(4-aminophenyl)-3-morpholinone.

This synthesis uses toxic solvents or reagents, which is disadvantageous per se, and in addition these toxic substances must be removed from the final product for regulatory reasons, which signifies additional expense. According to the description the product is obtained by precipitation and filtration after cooling the reaction mixture of toluene/1-methyl-2-pyrolidone, further by washing with water and drying.

An alternative method for producing rivaroxaban is described in WO 2005/068456 (EP1720866), which relates to a process for preparing rivaroxaban by reacting (*S*)-4-(4-(5-(aminomethyl)-2-oxooxazolidin-3-yl)phenyl)morpholin-3-one in a form of hydrochloride salt with 5-chlorothiophene-2-carbonyl chloride, characterized in that the reaction is carried out in solvent selected from group of ether, alcohol, ketone and water or in a mixture thereof with use of an inorganic base. Example "d" describes the process which includes acetone, water and toluene as solvents and sodium carbonate as inorganic base. The obtained solvent-containing crude product is further recrystallized from acetic acid. Large volumes of organic solvents are needed to obtain rivaroxaban. Furthermore at the end of the process solvents are filtered off as a mixture, which is difficult to recover and can not be re-used in the process. Therefore, an enviromentally more friendly process is needed.

Patent application WO 2012/035057 refers to WO 2005/068456 and provides additional information (Comparative Example 1) about the purity of a product obtained by the described process. This patent application further describes two process impurities namely as compound A and compound B, which are illustrated by the following formulas:

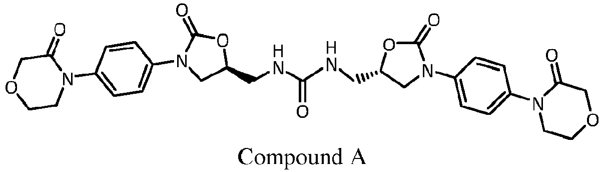

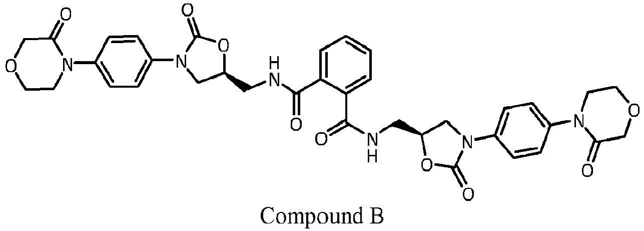

According to the Comparative Example the HPLC purity of rivaroxaban was 98.378 area %, compound A 0.023 w/w % and compound B 0.108 area %. However, this patent application does not provide any information about the other impurities.

A process which comprises reaction of the hereinafter defined compounds (II) and (III) is disclosed also in WO 2012/153155.

Therefore, there exist a need for an improved process for synthesis of rivaroxaban to be used on an industrial scale, whereby high yields and a high purity grade of the final product can be achieved in a significantly more simple way with less process steps, at lower temperatures, and which requires less solvent and does not require addition of solid inorganic base to the reaction mixture. Furthermore, so obtained mixtures of solvents after the completion of the reaction are easy to recover and can be re-used in the process.

Also there is need for an environmentally friendly process which uses small amounts of solvents and reagents, avoids hazardous solvents and reagents, and avoids mixtures of solvents and reagents as wastes of the process.

Like any synthetic compound, rivaroxaban can contain extraneous compounds or impurities that can come from many sources. These can include unreacted starting materials, by-products of the reaction, products of side reactions, or degradation products. Impurities in rivaroxaban or any active pharmaceutical ingredient (API) are undesirable, and, in extreme cases, might even be harmful to a patient being treated with a dosage form of the API in which a sufficient amount of impurities is present. It is also known in the art that impurities in an API may arise from degradation of the API itself, which is related to the stability of the pure API during storage, and the manufacturing process, including the chemical synthesis.

Therefore, there is still a need for an improved process comprising reaction systems which provide rivaroxaban of high yields and desired quality.

### Description of the invention

The present invention relates to polymorphic form K of compound (I) (rivaroxaban) according to claims 1 to 3, a process of its preparation according to claims 4 to 8, its use for the formulation of a pharmaceutical dosage form according to claim 9 as well as a pharmaceutical dosage form comprising it according to claim 10.

A process for preparation of rivaroxaban of the formula (I) having desired quality properties may be characterized in that the compound of the formula (II) is reacted with the compound of the formula (III) in a reaction medium which comprises 1-methylimidazole.

A preferred embodiment of this process is illustrated by the following scheme:

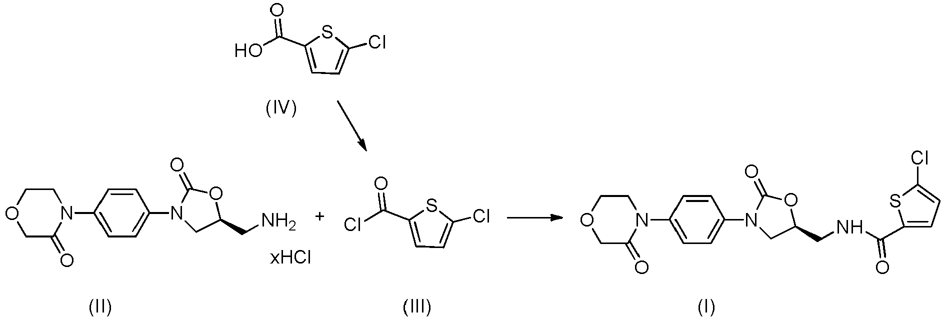

| | |
|---|---|
| Compound (I): | (S)-5-chloro-*N*-((2-oxo-3-(4-(3-oxomorpholino) phenyl) oxazolidin 5-yl) methyl) thiophene-2-carboxamide; Rivaroxaban |
| Compound (II): | (S)-4-(4-(5-(aminomethyl)-2-oxooxazolidin-3 yl)phenyl) morpholin-3-one hydrochloride |
| Compound (III): | 5-chlorothiophene-2-carbonyl chloride |
| Compound (IV): | 5-chlorothiophene-2-carboxylic acid |

In a first aspect, the process for preparing compound (I) comprises
(a) reaction of compound (II) with compound (III) in the presence of 1-methylimidazole.

In another aspect, the process for preparing compound (I) comprises
(a) reaction of compound (II) with compound (III) in the presence of 1-methylimidazole,
(b) optionally recovering compound (I) and
(c) optionally converting compound (I) into a desired crystalline form or salt form.

Compound (III) can be prepared from the compound (IV) as exemplified herein or according to the known processes as described for example in WO 2005/068456 and WO 2012/035057.

The temperature of the reaction is usually from -5°C to 30°C, preferably from 10°C to 25°C, more preferably at 17°C to 22°C.

The reaction time is usually from 0.25 to 4h, preferably from 0.5 to 2h, more preferably 1 hour.

It has surprisingly been found out that the reaction can take place at a high concentration of the intermediates, and therefore low amounts of the solvents are needed. The concentration of compound (II) in 1-methylimidazole at the beginning of the reaction is usually more than 0.5M, preferably between 0.7M and 2 M and most preferably between 0.8 and 1.2M.

The process is preferably carried out without addition of inorganic base.

After the completion of the reaction, compound (I) or a salt thereof is optionally recovered from the reaction mixture.

In a preferred embodiment compound (I) or a salt thereof is recovered by precipitation which is initiated with water, followed by the filtration.

The volume ratio of water which is added per volume 1-methylimidazole to initiate precipitation is usually less than 60 : 1, preferably less than 40 : 1, more preferably between 5 and 30 : 1.

The yield of the recovered compound (I) is usually more than 90%, preferably more than 93%.

Used 1-methylimidazole can be easily regenerated from the filtrate due to the high difference between boiling point of water and 1-methylimidazole. Regeneration can be performed according to any process known in the art.

The present inventors have further determined the specific impurity 5-chloro-N-methylthiophene-2-carboxamide (Impurity V) formed during the process for preparation of rivaroxaban which comprises reaction between compound (II) and compound (III).

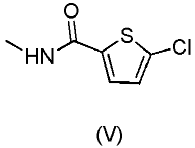

It has been surprisingly found out that compound (I) obtained according to the above process comprises less than 0.15 area %, preferably less than 0.10 area %, most preferably 0.07 area % of the impurity (V).

Rivaroxaban obtained by the above process can be obtained in different crystal forms or can be converted to different salt forms, which are known from the prior art which includes WO 2007/039132, WO 2009/149851, WO 2010/075631, WO 2012/004245, WO 2012/041263 and IPCOM000198340D.

The inventors of the present invention surprisingly found out that compound (I) can be crystallized in a new polymorphic form at low temperature.

It was surprisingly found out that polymorphic form K of compound (I) exists. The present invention provides the novel crystalline form as well as a process for preparing the same.

Compound (I) of polymorphic form K is characterized by the following significant peaks in its X-ray powder diffraction pattern:

| No. | Pos. [°2Th.] | Rel. Int. [%] |
|---|---|---|
| 1 | 3.6 | 43 |
| 2 | 7.1 | 22 |
| 3 | 14.3 | 22 |
| 4 | 19.9 | 92 |
| 5 | 20.2 | 63 |
| 6 | 24.3 | 100 |
| 7 | 28.9 | 39 |

Compound (I) of polymorphic K according to the present invention is prepared by a process which comprises the following steps:
a.) dissolving compound (I) in the presence of acetic acid,
b.) crystallization of compound (I) by addition of the solution to an anti-solvent, wherein the temperature of the crystallization medium is kept below 15°C, preferably from 0°C to 10°C, more preferably from 0°C to 5°C,
c.) optionally ageing and isolation of the crystals, and
d.) optionally drying the crystals.

Preferred anti-solvents comprise alkanes, esters end water. More preferred heptane, ethylacetate and water, most preferably water.

The processes of the invention can include an ageing step. The crystals which are formed upon cooling, can be kept at a low temperature within the above range for 0.01 hours to 300 hours, more preferably for 0.1 to 50 hours, even more preferably for 1 to 5 hours, and then isolated e.g. by filtration.

The crystals are preferably washed with anti-solvent and preferably dried under reduced pressure of 10 to 800 mbar to obtain compound (I) of polymorphic K. The drying temperature is preferably 20 ⁰C to 80 ⁰C, more preferably 30 ⁰C to 50 ⁰C.

Compound (I) of polymorphic form K of the present invention preferably has an average particle size in the range of 2 to 20 µm, particularly preferably 2 to 15 µm, and even more preferred 5 to 10 µm. The term "average particle size" or "particle size" as used herein refers to the volume mean diameter of particles.

The rivaroxaban can be further micronized to obtain particles with d₉₀ < 40 µm, more preferably d₉₀ < 20 µm, and most preferably d₉₀ < 15 µm. As used herein d₉₀ < x means that at least 90 % by volume of the particles have a particle size below x.

Micronized rivaroxaban can be obtained for instance by single or multi-stage micronization in the dry state using dry mills, such as cutting mills, pin/cage mills, hammer mills, jet mills, fluidized bed jet mills, ball mills and roller mills.

Compound (I) of polymorphic K exhibits advantageous physical and pharmacological properties. It is having a uniform and well-defined morphology, a favorable dissolution rate, bioavailability, inter-individual variability, and chemical stability. In particular it is evident, that polymorphic form K has a characteristic plate-like morphology of small particles. Plate-like morphology of form K compared to needle like morphology of form I has advantages in form of an improved milling processibility and better dissolution rates of polymorph form K. Therefore, compound (I) of polymorphic K is in particularly suitable when formulated to pharmaceutical dosage forms, such as tablets, capsules, pellets, granules, and powders.

In the following the invention will be described by use of examples. In the examples, the following analytical methods are used:
Chromatographic purity was measured on a X-Bridge Shield RP18 Column (3.5 µm, 150x4.6 mm), mobile phase A.) 0.01M NH₄H₂PO₄ pH 5.0; B.) 90 % Acetonitrile, Gradient: 0 min - 9 %B, 8 min - 23 %B, 25 min - 50 %B, 28-29 min - 100 %B, 32 min - 9 %B; The chromatograph was equipped with a UV detector set at 250 nm, flow rate was 1.0 ml/min at 25°C. Results are expressed as area %.

Water content was determined by Karl Fisher method as described in Ph.Eur. 2.5.12.

X-ray powder diffraction patterns reported herein were obtained by a PANalytical X'Pert PRO MPD diffractometer using CuK_{α} radiation, 1.54178 A, 3°<2θ<30°.

The term "Theta" (Θ) is the angle between the atomic plane and both the incident and reflected beams.

The particle size was determined by laser light scattering method using a Malvern Mastersizer Apparatus MS 2000 equipped with a Hydro G dispersion unit using purified water as the dilution medium.

### Examples:

### Example 1:

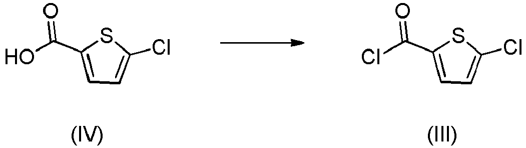

6,4 g (19,7 mmol) of compound (IV), 30 ml of methylene chloride (CH₂Cl₂), 20 ml thionyl chloride (SOCl₂) and 50 µl of N,N-dimethylformamide (DMF) were charged to a 250 ml flask. The reaction mixture was heated to reflux temperature and stirred at this temperature for 16 hrs. The obtained mixture was further evaporated, dried 5 times with 15 ml of toluene and further 2 times with 15 ml of CH₂Cl₂. An amount of 6.25 g of compound (III) was obtained (Yield 92 %).

### Example 2:

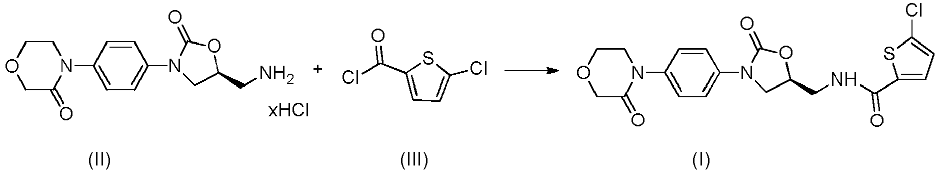

14.1 ml of 1-methylimidazole and 4.66 g (14,2 mmol) of compound (II) were charged to a 50 ml flask.

The solution was cooled to 0 °C, and then 3.26 g (18 mmol) of compound (III) were added dropwise.

The reaction mixture was heated to room temperature and stirred for 1 hour. A sample was taken after 30 min of stirring. After one hour 400 ml of demineralised water were added to initiate precipitation. The mixture was further stirred for 3 hours, and then the product was filtered, rinsed with demineralised water and dried.

An amount of 6.27 g of compound (I) was obtained, having a water content of 7.2 % determined by Karl Fischer (Yield including water: 101.2 %, yield when subtracting water: 94 %). The chromatographic purity of compound (I) was 99.30 area %, impurity (V) 0.06 area %, both determined by HPLC.

### Reference example A

Comparative example 1 according to document WO 2012/035057 was repeated exactly as described.

The chromatographic purity of the compound (I) and the amount of impurity (V) as obtained by the comparative example, as well as the yield are compared with the product according to the Example 2 in the following table:

| | Reference example A | Example 2 |
|---|---|---|
| Yield | 93.8 % | 94 % |
| Chromatographic purity of compound (I) | 99.01 area % | 99.30 area % |
| Rt 19.049 min | | |
| Impurity (V) | 0.40 area % | 0.06 area % |
| Rt 14.742 min | | |

### Example 3 (comparative)

31.02 g of compound (I) and 465,3 ml of acetic acid were charged in a 1 l flask and heated to reflux temperature (compound (I) was dissolved before the reflux temperature is reached). The clear hot solution was filtered and slowly added to 413 ml demineralised water at a temperature between 20 and 35 °C. Precipitated product was stirred for another 3 hours at the same temperature, then filtered, and the product was rinsed with 103 ml of demineralised water and dried in an air drier at 40 °C. The so obtained product (27.37 g) was characterized as polymorphic form 1 as defined in WO 2007/039132 determined by XRPD.

### Example 4

Compound (I) (2.5 g) was dissolved in acetic acid (35 mL) at 80 °C. The solution was filtered through a 1 µm filter, and the filter was washed with 2.5 mL of acetic acid. The hot solution was dropwise added into cold water (33.5 mL) in a reactor with an overhead stirrer. During the addition the temperature in the reactor was maintained around 5 °C. Crystallized product was stirred at 5 °C for 2 hours and collected by filtration. The final product was washed with water and dried in a vacuum drier at 50 °C. The obtained product (2.2 g) was characterized as polymorphic form K by XRPD that has characteristic platted morphology. The HPLC purity of the product was 99.8%. The average particle size of the product was 17.2 µm, d₉₀ < 30 µm.

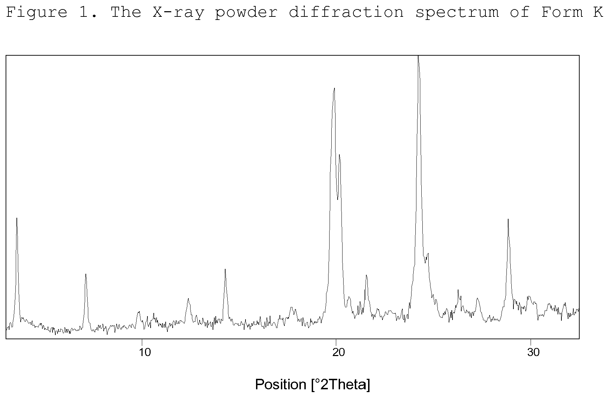

The X-ray powder diffraction spectrum of Form K is shown in Figure 1.

### Example 5

Compound (I) (5 g) was dissolved in acetic acid (75 mL) at 80 °C. The solution was filtered through a 1 µm filter. The hot solution was dropwise added into cold water (66.5 mL) in a reactor with an overhead stirrer. During the addition the temperature in the reactor was maintained around 5 °C. Crystallized product was stirred at 5 °C for 2 hours and approximately half of the suspension was collected by filtration. The product was washed with water and dried in a vacuum drier at 50 °C. The so obtained product (1.6 g) was characterized as polymorphic form K by XRPD that has characteristic platted morphology. The remaining suspension in the reactor was heated to 20 °C and stirred for 15 hours. The suspension was collected by filtration. The product was washed with water and dried in a vacuum drier at 50 °C. The so obtained product (2.2 g) was characterized as polymorphic form 1 as defined in WO 2007/039132 determined by XRPD that has characteristic needle

like morphology.

## Claims

1. Compound (I) of polymorphic form K 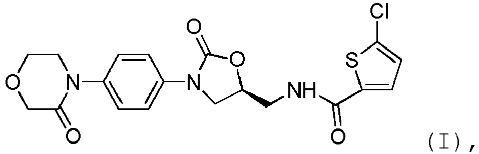 **characterized by** the following significant peaks in its X-ray powder diffraction pattern:
| No. | Position [°2Theta] | Relative Intensitiy [%] |
|---|---|---|
| 1 | 3.6 | 43 |
| 2 | 7.1 | 22 |
| 3 | 14.3 | 22 |
| 4 | 19.9 | 92 |
| 5 | 20.2 | 63 |
| 6 | 24.3 | 100 |
| 7 | 28.9 | 39 |

2. Compound according to claim 1 having an average particle size in the range of 2 to 20 µm, preferably 2 to 15 µm, more preferably 5 to 10 µm.

3. Compound according to claim 1 having a d₉₀ < 40 µm, more preferably a d₉₀ < 20 µm, and most preferably a d₉₀ < 15 µm.

4. Process for preparing the compound according to claim 1 wherein the process comprises the following steps:
a.) dissolving compound (I) in the presence of acetic acid,
b.) crystallization of compound (I) by addition of the solution to an anti-solvent, wherein the temperature of the crystallization medium is kept below 15°C, preferably from 0°C to 10°C, more preferably from 0°C to 5°C,
c.) optionally ageing and isolation of the crystals, and
d.) optionally drying the crystals.

5. Process according to claim 4, wherein the anti-solvent is selected from alkanes, esters and water.

6. Process according to claim 5, wherein the anti-solvent is selected from heptane, ethylacetate and water, and preferably is water.

7. Process according to any one of claims 4 to 6, wherein the aging is carried out by keeping the crystals formed upon cooling in step b.) below 15°C, preferably from 0°C to 10°C, more preferably from 0°C to 5°C, for 0.01 to 300 hours, preferably 0.1 to 50 hours, more preferably 1 to 5 hours.

8. Process according to any one of claims 4 to 7, wherein the drying is carried out at a drying temperature of 20 to 80 °C, preferably 30 to 50°C.

9. Use of the compound according to any one of claims 1 to 3 for the formulation of a pharmaceutical dosage form, such as a tablet, capsule, pellet, granule or powder.

10. Pharmaceutical dosage form comprising the compound according to any one of claims 1 to 3.

## Patentansprüche

1. Verbindung (I) der polymorphen Form K 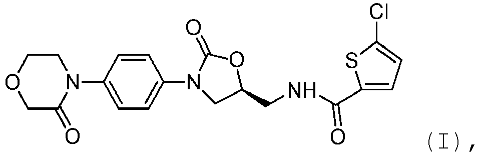 die anhand der folgenden signifikanten Peaks in ihrem Röntgenpulverdiffraktogramm gekennzeichnet ist:
| Nr. | Position [2θ,°] | Relative Intensität [%] |
|---|---|---|
| 1 | 3, 6 | 43 |
| 2 | 7,1 | 22 |
| 3 | 14,3 | 22 |
| 4 | 19,9 | 92 |
| 5 | 20,2 | 63 |
| 6 | 24,3 | 100 |
| 7 | 28,9 | 39 |

2. Verbindung nach Anspruch 1 mit einer mittleren Teilchengröße von 2 bis 20 µm, bevorzugt von 2 bis 15 µm und besonders bevorzugt von 5 bis 10 µm.

3. Verbindung nach Anspruch 1 mit einer d₉₀ < 40 µm, bevorzugt mit einer d₉₀ < 20 µm und besonders bevorzugt mit einer d₉₀ < 15 µm.

4. Verfahren zur Herstellung der Verbindung nach Anspruch 1, bei dem
a.) Verbindung (I) in Gegenwart von Essigsäuse aufgelöst wird,
b.) Verbindung (I) durch Zugabe der Lösung eines Antilösungsmittels kristallisiert, wobei die Temperatur des Kristallisationsmediums unter 15 °C, bevorzugt im Bereich von 0 bis 10 °C und besonders bevorzugt im Bereich von 0 bis 5 °C, gehalten wird,
c.) die Kristalle gegebenenfalls gealtert und isoliert werden und
d.) die Kristalle gegebenenfalls getrocknet werden.

5. Verfahren nach Anspruch 4, bei dem das Antilösungsmittel aus Alkanen, Estern und Wasser ausgewählt ist.

6. Verfahren nach Anspruch 5, bei dem das Antilösungsmittel aus Heptan, Ethylacetat und Wasser ausgewählt ist, und vorzugsweise Wasser ist.

7. Verfahren nach einem der Ansprüche 4 bis 6, bei dem die Alterung durchgeführt wird, indem die während Schritt b.) gebildeten Kristalle über 0,01 bis 300 Stunden, bevorzugt 0,1 bis 50 Stunden und besonders bevorzugt 1 bis 5 Stunden, bei unter 15 °C, bevorzugt im Bereich von 0 bis 10°C und besonders bevorzugt im Bereich von 0 bis 5 °C, gehalten werden.

8. Verfahren nach einem der Ansprüche 4 bis 7, bei dem die Trocknung bei einer Trocknungstemperatur von 20 bis 80 °C, bevorzugt von 30 bis 50 °C, durchgeführt wird.

9. Verwendung der Verbindung nach einem der Ansprüche 1 bis 3 zur Zubereitung einer pharmazeutischen Dosierungsform, wie Tablette, Kapsel, Pellet, Granulat oder Puder.

10. Pharmazeutische Dosierungsform, welche die Verbindung nach einem der Ansprüche 1 bis 3 enthält.

## Revendications

1. Composé (I) de forme polymorphe K 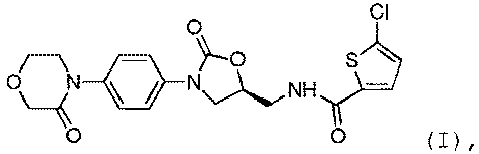 **caractérisé par** les pics significatifs suivants dans son diagramme de diffraction des rayons X à l'état de poudre :
| Numéro | Position [2θ, °] | Intensité relative [%] |
|---|---|---|
| 1 | 3,6 | 43 |
| 2 | 7,1 | 22 |
| 3 | 14,3 | 22 |
| 4 | 19,9 | 92 |
| 5 | 20,2 | 63 |
| 6 | 24,3 | 100 |
| 7 | 28,9 | 39 |

2. Composé selon la revendication 1, présentant une taille moyenne de particules située dans l'intervalle allant de 2 à 20 µm, de préférence de 2 à 15 µm, et mieux encore de 5 à 10 µm.

3. Composé selon la revendication 1, présentant une taille d₉₀ de moins de 40 µm, de préférence une taille d₉₀ de moins de 20 µm, et mieux encore une taille d₉₀ de moins de 15 µm.

4. Procédé de préparation du composé selon la revendication 1, dans lequel le procédé comprend les étapes suivantes :
a.) dissoudre du composé (I) en présence d'acide acétique,
b.) faire cristalliser le composé (I) en ajoutant la solution dans un non-solvant, dans lequel la température du milieu de cristallisation est maintenue au-dessous de 15°C, et de préférence à une valeur de 0°C à 10°C, et mieux encore de 0°C à 5°C,
c.) en option, laisser vieillir les cristaux et les isoler, et
d.) en option, faire sécher les cristaux.

5. Procédé selon la revendication 4, dans lequel le non-solvant est choisi parmi des alcanes, des esters et de l'eau.

6. Procédé selon la revendication 5, dans lequel le non-solvant est choisi parmi de l'heptane, de l'acétate d'éthyle et de l'eau, mais est de préférence de l'eau.

7. Procédé selon l'une quelconque des revendications 4 à 6, dans lequel le vieillissement est réalisé en maintenant les cristaux formés lors du refroidissement à l'étape b.) au-dessous de 15°C, de préférence de 0°C à 10°C, et mieux encore de 0°C à 5°C, pendant 0,01 à 300 heures, de préférence de 0,1 à 50 heures, et mieux encore de 1 à 5 heures.

8. Procédé selon l'une quelconque des revendications 4 à 7, dans lequel le séchage est effectué à une température de séchage de 20 à 80°C, et mieux encore de 30 à 50°C.

9. Utilisation du composé selon l'une quelconque des revendications 1 à 3 pour la formulation d'une forme galénique, comme un comprimé, une gélule, une pastille, un granulé ou une poudre.

10. Forme galénique comprenant le composé selon l'une quelconque des revendications 1 à 3.
